(19) Europäisches Patentamt
European Patent Office
Office européen des brevets

(11) **EP 4 671 238 A1**

(12) **EUROPEAN PATENT APPLICATION**
published in accordance with Art. 153(4) EPC

(43) Date of publication:
**31.12.2025 Bulletin 2026/01**

(21) Application number: **24746175.9**

(22) Date of filing: **12.01.2024**

(51) International Patent Classification (IPC):
***C07D 295/13*** (2006.01) ***A61K 9/51*** (2006.01)
***A61K 9/127*** (2025.01) ***A61K 47/22*** (2006.01)
***A61K 48/00*** (2006.01)

(52) Cooperative Patent Classification (CPC):
**C07D 295/13; A61K 31/7105; A61K 31/713;**
A61K 9/0019; A61K 9/5123; A61K 38/00

(86) International application number:
**PCT/KR2024/000628**

(87) International publication number:
**WO 2024/177282 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.02.2023 KR 20230023894**

(71) Applicant: **SurgiNex Co., Ltd.**
**Seoul 06591 (KR)**

(72) Inventors:
- **LEE, Hyukjin**
  **Seoul 06557 (KR)**
- **KIM, Minjeong**
  **Seoul 05119 (KR)**
- **JEONG, Michaela**
  **Seoul 05307 (KR)**
- **LEE, Yeji**
  **Seongnam-si, Gyeonggi-do 13643 (KR)**
- **PARK, Jeongeun**
  **Seoul 05307 (KR)**
- **JUNG, Hyein**
  **Seoul 05090 (KR)**
- **IM, Seongeun**
  **Seoul 03766 (KR)**

(74) Representative: **Patentanwaltskanzlei Matschnig & Forsthuber OG**
**Biberstraße 22**
**Postfach 36**
**1010 Wien (AT)**

(54) **IONIZABLE LIPID COMPRISING SULFIDE, AND LIPID NANOPARTICLES COMPRISING SAME**

(57) The disclosure relates to a novel sulfide-containing ionizable lipid. The ionizable lipid of the disclosure interacts electrostatically with an anionic drug when a lipid nanoparticle is produced, thereby ensuring that the drug is encapsulated in the lipid nanoparticle with high efficiency and the drug is stably delivered into the body, facilitating its utility in the related art such as lipid nanoparticle-mediated gene therapy.

FIG. 6

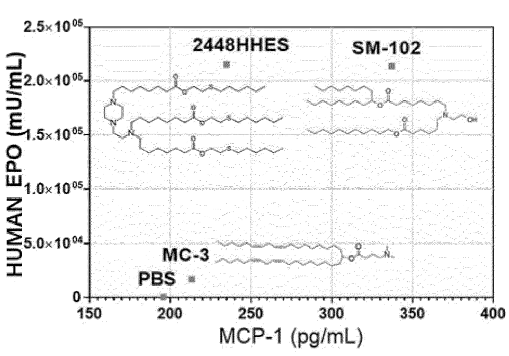


EP 4 671 238 A1

## Description

### [Technical Field]

The disclosure relates to a sulfide-containing ionizable lipid, and specifically, to a sulfide-containing ionizable lipid, a lipid nanoparticle produced using the same, and use thereof.

### [Background Art]

A drug delivery system (DDS) is a technology designed to efficiently deliver the necessary amount of drug while minimizing side effects of the drug and maximizing efficacy and effectiveness. In particular, in gene therapy, viral vectors have been proven effective as drug delivery vehicles; however, the use of viruses as gene delivery systems is limited due to various drawbacks such as immunogenicity, size limitations of the injected DNA, and challenges in mass production.

Accordingly, as alternatives to viral systems, methods for delivering nucleic acids into cells typically involve mixing nucleic acids with cationic lipids or polymers (respectively referred to as lipid-DNA conjugates (lipoplexes) and polymer-DNA conjugates (polyplexes)) (Hirko et al., Curr, Med, Chem., 10, 1185-1193, 2003; Merdan et al., Adv. Drug. Deliv.Rev., 54, 715-758, 2002; Spagnou et al., Biochemistry, 43, 13348-13386, 2004). In particular, lipid-DNA conjugates are widely used at the cellular level because they bind to nucleic acids and deliver nucleic acids well into cells; however, there are drawbacks in that when injected locally in vivo, they often cause inflammation within the body (Filonand and Phillips, Biochim. Biophys/Acta, 1329, 345-356, 1997), and when injected into a blood vessel, they accumulate in tissues such as the lungs, liver, spleen, etc., which are organs through which they primarily transit (Ren et al., Gene Therapy. 7, 764-768, 2000).

### [Disclosure]

### [Technical Problem]

The inventors have made diligent efforts to develop a novel material that has an excellent drug encapsulation rate and is capable of efficiently deliver anionic drugs such as nucleic acids to target organs or cells. As a result, the inventors have completed the disclosure by confirming excellent drug delivery effects of a novel sulfide-containing ionizable lipid of the disclosure.

### [Technical Solution]

One objective of the disclosure is to provide a sulfide-containing ionizable lipid having a novel structure, a stereoisomer thereof, or a pharmaceutically acceptable salt thereof.

Another objective of the disclosure is to provide a lipid nanoparticle including the ionizable lipid, the stereoisomer thereof, or the pharmaceutically acceptable salt thereof.

Another objective of the disclosure is to provide a drug delivery composition that includes the lipid nanoparticle and an anionic drug and delivers the drug specifically to the liver or lungs.

### [Advantageous Effects]

A sulfide-containing ionizable lipid of the disclosure interacts electrostatically with an anionic drug when a lipid nanoparticle is produced, thereby ensuring that the drug is encapsulated in the lipid nanoparticle with high efficiency and the drug is specifically and stably delivered to the liver or lungs.

### [Description of Drawings]

FIG. 1 is an in vitro efficacy test and shows results of measuring the luminescence intensity of mRNA-encapsulating lipid nanoparticles including ionizable lipids of the disclosure in Hela, HepG2, HEK 239, and MRC-5 cells.

FIG. 2 is an in vivo efficacy test and shows photographs showing bioluminescence after intravenous injection into a mouse, to confirm the in vivo delivery of mRNA-encapsulating HHES lipid nanoparticles according to the type of ionizable lipid.

FIG. 3 is a graph confirming and comparing bioluminescence in the liver after intravenous injection into a mouse, to confirm the in vivo delivery efficacy of mRNA-encapsulating HHES lipid nanoparticles according to the type of

ionizable lipid.

FIG. 4 is an in vivo efficacy test and shows a photograph showing bioluminescence after intramuscular injection into a mouse, to confirm the in vivo delivery of mRNA-encapsulating HHES lipid nanoparticles according to the type of ionizable lipid.

FIG. 5 is a graph confirming and comparing bioluminescence after intramuscular injection into a mouse, to confirm the in vivo delivery efficacy of mRNA-encapsulating HHES lipid nanoparticles according to the type of ionizable lipid.

FIG. 6 is a graph confirming the level of EPO protein in the blood and the level of MCP-1 after intravenous injection into a mouse, to confirm the in vivo delivery efficacy and initial immunogenicity of hEPO mRNA-encapsulating lipid nanoparticles.

FIG. 7 is an in vivo efficacy test and shows photographs confirming bioluminescence after intravenous injection into a mouse, to confirm the in vivo delivery of mRNA-encapsulating lipid nanoparticles according to the proportion of DOTAP included.

FIG. 8 is a graph confirming and comparing bioluminescence in the liver, spleen, and lungs after intravenous injection into a mouse, to confirm the in vivo delivery efficacy of mRNA-encapsulating lipid nanoparticles according to the proportion of DOTAP included.

FIG. 9 is a graph confirming and comparing the distribution of luminescence in each organ after intravenous injection into a mouse, to confirm the in vivo delivery efficacy of mRNA-encapsulating lipid nanoparticles according to the proportion of DOTAP included.

**[Best Mode]**

The detailed description is provided as follows. Meanwhile, the descriptions and embodiments disclosed in the disclosure may also be applied to other descriptions and embodiments. In other words, all combinations of various elements disclosed in the disclosure fall within the scope of the disclosure. In addition, it is not considered that the scope of the disclosure is limited by the detailed description provided below.

As a result of research and efforts by the inventors to achieve the above objectives, they have completed a sulfide-containing ionizable lipid represented by Chemical Formula 1 below and a lipid nanoparticle including the same. In addition, it has been confirmed that the lipid nanoparticle stably and effectively delivers a drug, for example, a nucleic acid, to the liver or lungs.

**Sulfide-containing ionizable lipid**

To achieve the above objectives, one aspect of the disclosure relates to an ionizable lipid represented by Chemical Formula 1 below, a stereoisomer thereof, or a pharmaceutically acceptable salt thereof.

[Chemical Formula 1]

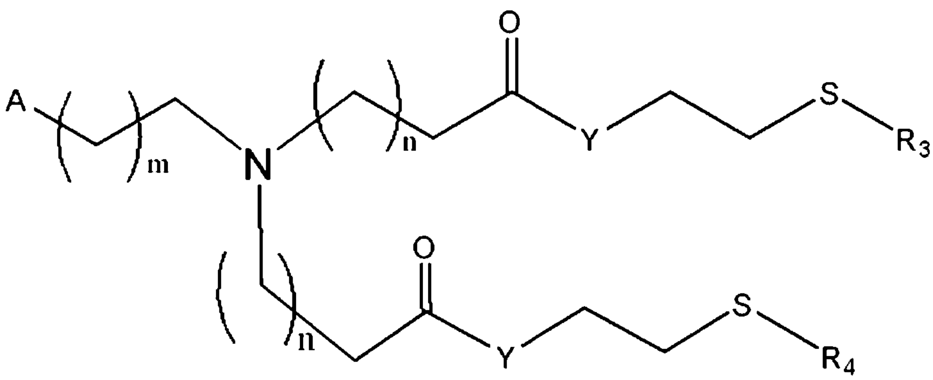

In Chemical Formula 1,

A is

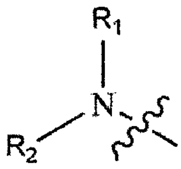

or

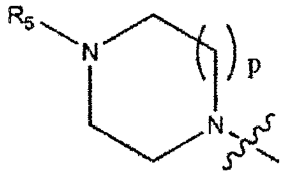

,

$R_1$ and $R_2$ are each independently any one selected from -H, -$C_{1-6}$alkyl, -$C_{1-6}$alkyl-$NR_3R_4$, and-$C_{2-12}$alkyl-(C=O)-Y-$(CH_2)_x$S-$C_{4-12}$alkyl,
$R_3$ and $R_4$ are each independently -$C_{2-8}$alkyl,
$R_5$ is -$C_{2-12}$alkyl-(C=O)-Y-$(CH_2)_x$S-$C_{4-12}$alkyl,
Y is -O- or $NR_6$,
$R_6$ is -H or -$C_{1-3}$alkyl,
m is an integer from 0 to 3,
n is independently an integer from 2 to 7,
x is an integer from 1 to 3, and
p is an integer from 0 to 2.

The disclosure is not limited thereto, but in a compound represented by Chemical Formula 1, specifically, in Chemical Formula 1,

A may be

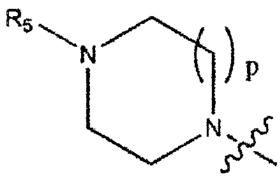

,

$R_5$ may be -$C_{2-12}$alkyl-(C=O)-Y-$(CH_2)_x$S-$C_{4-12}$alkyl,
Y may be -O-,
m may be an integer from 0 to 3,
n may independently be an integer from 2 to 7,
x may be an integer from 1 to 3, and
p may be 1.

In addition, according to a detailed example of the disclosure, the compound represented by Chemical Formula 1 may include at least any one selected from the group consisting of compounds described in Table 1 below.

[Table 1]

| Compound | Structure |
|---|---|
| 1 | 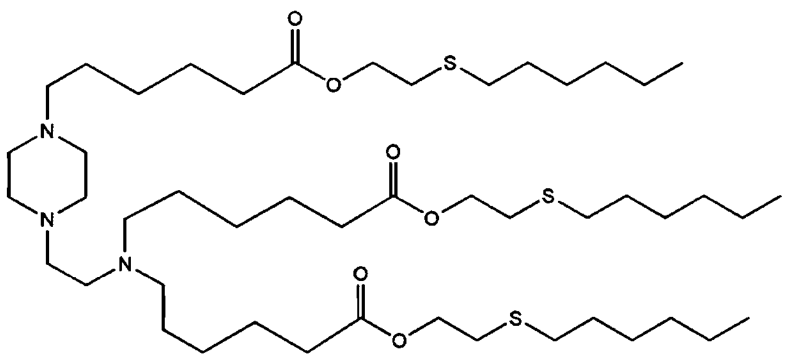 |

(continued)

| Compound | Structure |
|---|---|
| 2 | 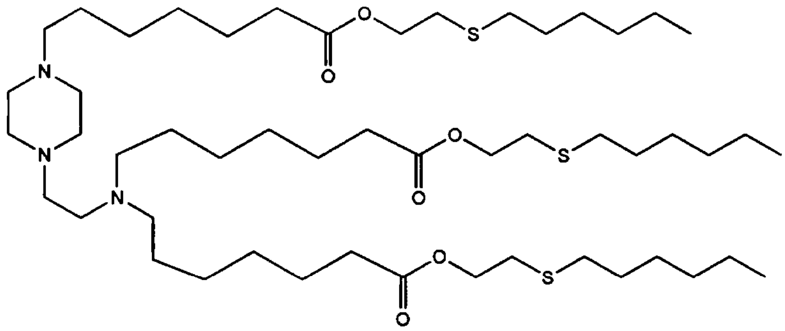 |
| 3 | 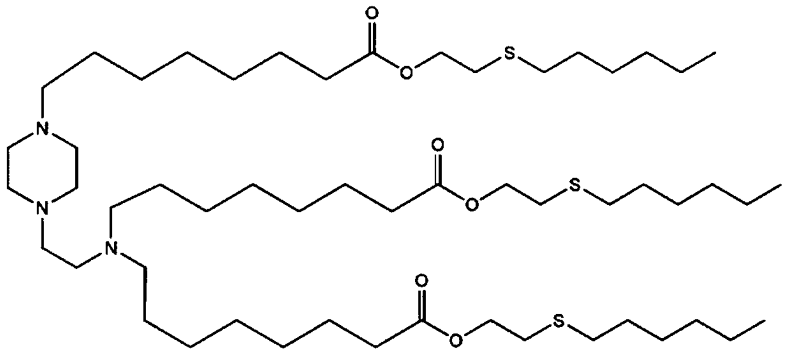 |
| 4 | 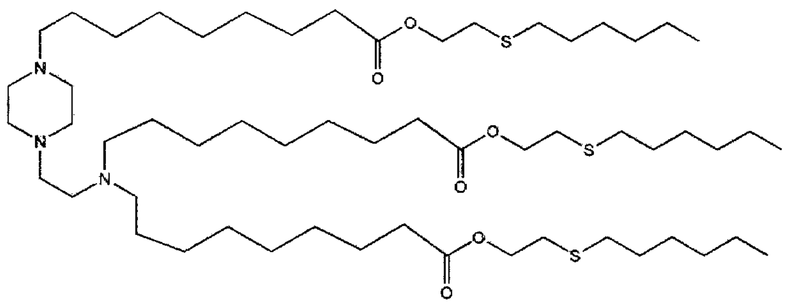 |

In the disclosure, unless otherwise specified, the term "alkyl" refers to a linear or branched acyclic saturated hydrocarbon. For example, "$C_{1-6}$alkyl" may refer to alkyl containing one to six carbon atoms. Even in a case where a simple substituent is added to an alkyl structure of the disclosure, as long as it has the same effect as the ionizable lipid of the disclosure, it is included in the scope of the disclosure to the extent of equivalents.

In the disclosure, "ionizable lipid" refers to an amine-containing lipid that may be easily protonated, and is also referred to as a lipidoid, and for example, may be a lipid of which a charge state changes depending on the surrounding pH. It plays a role in ensuring that the drug is encapsulated within lipid nanoparticles with high efficiency through electrostatic interaction with anionic drugs, and contributes to forming the structure of lipid nanoparticles. The ionizable lipid of the disclosure is characterized in that a sulfide-containing alkyl chain is bonded to an amine-containing head portion. Specifically, the ionizable lipid may be a compound having properties similar to lipids produced by reaction of an alkyl-sulfide and an amine including a piperazine structure.

The ionizable lipid is an ionizable compound having properties similar to lipids, and through electrostatic interaction with drugs (for example, anionic drugs and/or nucleic acids), allows the drugs to be encapsulated within lipid nanoparticles with high efficiency.

In the disclosure, the term "stereoisomer" refers to compounds of the disclosure, which have the same chemical formula or molecular formula but differ sterically. Each stereoisomer and mixtures thereof are also included within the scope of the disclosure. Unless otherwise specified, the solid line bond (-) connected to an asymmetric carbon atom may include a wedge-shaped solid line bond (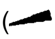) or a wedge-shaped dashed line bond (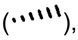), indicating the absolute arrangement of the stereocenter.

The compound of Chemical Formula 1 of the disclosure may exist in the form of a "pharmaceutically acceptable

salt." As a salt, an acid addition salt formed by a pharmaceutically acceptable free acid may be useful, but the disclosure is not limited thereto. The term "pharmaceutically acceptable salt" of the disclosure has a concentration that has a significant action that is relatively non-toxic and harmless to patients, and refers to any organic or inorganic acid addition salt or base addition salt of the compound represented by Chemical Formula 1, wherein the side effects due to the salt do not diminish the beneficial effects of the compound.

An acid addition salt may be produced by common methods, for example, by dissolving the compound in an excess of aqueous acid solution and precipitating the salt using a water-miscible organic solvent, such as methanol, ethanol, acetone, or acetonitrile. Equimolar amounts of the compound and an acid or alcohol in water may be heated, and the mixture may then be evaporated to dryness, or the precipitated salt may be filtered off by suction.

At this time, the free acid may include an organic acid and an inorganic acid, wherein the inorganic acid may include hydrochloric acid, phosphoric acid, sulfuric acid, or nitric acid, and the organic acid may include methanesulfonic acid, p-toluenesulfonic acid, acetic acid, trifluoroacetic acid, maleic acid, succinic acid, oxalic acid, benzoic acid, tartaric acid, fumaric acid, manderic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, or hydroiodic acid, but the disclosure is not limited thereto.

In addition, a pharmaceutically acceptable metal salt may be produced using a base. An alkali metal salt or an alkaline earth metal salt may be obtained, for example, by dissolving the compound in an excess of alkali metal hydroxide or alkaline earth metal hydroxide solution, filtering the insoluble compound salt, and then evaporating and drying the filtrate. At this time, it is possible to produce a metal salt, for example, sodium, potassium, or a calcium salt, but the disclosure is not limited thereto. In addition, a corresponding silver salt may be obtained by reacting an appropriate silver salt (for example, silver nitrate) with an alkali metal or an alkaline earth metal salt.

**Lipid nanoparticle including sulfide-containing ionizable lipid**

Another aspect of the disclosure relates to a lipid nanoparticle including the ionizable lipid represented by Chemical Formula 1, the stereoisomer thereof, or the pharmaceutically acceptable salt thereof. The lipid nanoparticle of the disclosure may include at least one ionizable lipid represented by Chemical Formula 1. In addition, depending on the objective, an ionizable lipid other than that of the disclosure may be further included.

The lipid nanoparticle may further include at least any one selected from the group consisting of a helper lipid, a structural lipid, and a PEG-lipid, but the disclosure is not limited thereto.

The helper lipid that may promote fusion of lipid nanoparticles may be used without limitation, and may be, for example, dioleoylphosphatidylethanolamine (DOPE), distearoylphosphatidylcholine (DSPC), palmitoyloleoylphosphatidylcholine (POPC), egg phosphatidylcholine (EPC), dioleoylphosphatidylcholine (DOPC), dipalmitoylphosphatidylcholine (DPPC), dioleoylphosphatidylglycerol (DOPG), dipalmitoylphosphatidylglycerol (DPPG), distearoylphosphatidylethanolamine (DSPE), 1,2-dioleoyl-3-trimethylammonium-propane (DOTAP), phosphatidylethanolamine (PE), dipalmitoylphosphatidylethanolamine, 1,2-dioleoyl-sn-glycero-3-phosphate (18-PA), 1,2-dilinoleoyl-sn-glycero-3-phosphocholine, 1,2-diarachidoyl-sn-glycero-3-phosphocholine, 1,2-didocosahexaenoyl-sn-glycero-3-phosphocholine, 1,2-diphytanoyl-sn-glycero-3-phosphoethanolamine (ME 16:0 PE), 1-palmitoyl-2-oleoyl-sn-glycero-3-phosphoethanolamine (POPE), 1,2-dioleoyl-sn-glycero-3-[phospho-L-serine] (DOPS), 1,2-dilinoleoyl-sn-glycero-3-phosphocholine (DLPC), 1,2-dimyristoyl-sn-glycero-3-phosphocholine (DMPC), 1,2-diundecanoyl-sn-glycero-phosphocholine (DUPC), 1,2-di-O-octadecenyl-sn-glycero-3-phosphocholine (18:0 diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-distearoyl-sn-glycero-3-phosphoethanolamine, 1,2-dilinoleoyl-sn-glycero-3-phosphoethanolamine, 1,2-dilinoleoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, sphingomyelin, or a mixture thereof.

In the disclosure, PEG-lipid refers to a conjugated form of lipid and PEG, and refers to a lipid to which polyethylene glycol polymer, which is a hydrophilic polymer, is bound. The PEG-lipid contributes to particle stability in serum of nanoparticles within lipid nanoparticles and prevents aggregation between nanoparticles. In addition, the PEG-lipid protects nucleic acids from degradation enzymes, thereby enhancing the stability of nucleic acids in the body, and increases the half-life of drugs encapsulated in nanoparticles. The PEG-lipid may be, for example, PEG-ceramide, PEG-DMG, PEG-c-DOMG, PEG-DLPE, PEG-DMPE, PEG-DPPC, PEG-DSPE, or a mixture thereof, but the disclosure is not limited thereto.

The structural lipid maintains the particle shape within lipid nanoparticles and is dispersed on the core and surface of the nanoparticles to improve the stability of the nanoparticles. The structural lipid may be cholesterol, cholestenol, spinasterol, fecosterol, sitosterol, ergosterol, ergostenol, campesterol, stigmasterol, brassicasterol, tomatidine, ursolic acid, alpha-tocopherol, or a mixture thereof, but the disclosure is not limited thereto.

As a specific example, when the ionizable lipid of the disclosure, a helper lipid, a structural lipid, and a PEG-lipid are mixed to produce a lipid nanoparticle, the molar ratio of the ionizable lipid:the helper lipid:the structural lipid:the PEG-lipid may be 10 to 40:10 to 70:40 to 70:1 to 5. In addition, the molar ratio may be 15 to 35:10 to 30:45 to 65:1 to 4 or 20 to 30:15 to 25:50 to 60:1 to 3, but the disclosure is not limited thereto.

The lipid nanoparticle of the disclosure exhibits a positive charge under acidic pH conditions, and thus, easily forms a complex with drugs through electrostatic interaction with therapeutic agents, for example, nucleic acids that exhibit a negative charge and anionic drugs, to thereby encapsulate anionic drugs with high efficiency, and may be used as a composition for intracellular or in vivo drug delivery. Therefore, the lipid nanoparticle of the disclosure may be useful for the delivery of not only nucleic acids but also all types of drugs with anionic properties. In other words, the lipid nanoparticle of the disclosure may ultimately be produced in a form (encapsulated form) that further includes an anionic drug.

In the disclosure, the term "encapsulation" refers to encapsulating a delivery substance to enclose it and allow it to enter the body efficiently, and "drug encapsulation rate (encapsulation efficiency)" refers to the amount of drug encapsulated in lipid nanoparticles relative to the total amount of drug used for production.

The anionic drug may be a nucleic acid, a low-molecular weight compound, a peptide, a protein, a protein-nucleic acid structure, or an anionic biopolymer-drug conjugate, but as long as it may be stably and efficiently delivered by forming a lipid nanoparticle with the ionizable lipid of the disclosure, the disclosure is not limited thereto.

In the disclosure, the nucleic acid may be small interfering ribonucleic acid (siRNA), ribosomal ribonucleic acid (rRNA), deoxyribonucleic acid (DNA), complementary deoxyribonucleic acid (cDNA), aptamer, messenger ribonucleic acid (mRNA), transport ribonucleic acid (tRNA), sgRNA, antisense oligonucleotide, shRNA, miRNA, ribozyme, PNA, DNAzyme, or a mixture thereof, but the disclosure is not limited thereto.

The weight ratio of total lipid/nucleic acid m the lipid nanoparticle may be 1 to 20, specifically, 5 to 15, and more specifically, 7 to 12, but the disclosure is not limited thereto.

In the disclosure, the lipid nanoparticle may have a diameter of, for example, 40 nm to 150 nm, specifically, 50 nm to 140 nm, and more specifically, 60 nm to 130 nm, but the disclosure is not limited thereto.

Another aspect of the disclosure, the lipid nanoparticle may further include 1,2-dioleoyl-3-trimethylammonium-propane (DOTAP) as a helper lipid.

As a specific example, when the ionizable lipid of the disclosure, DOTAP, a structural lipid, and a PEG-lipid are mixed to produce a lipid nanoparticle, the DOTAP may be included in the total lipid of the lipid nanoparticle at 10 mol% to 70 mol%. Specifically, the molar ratio of the ionizable lipid:the DOTAP:the structural lipid:the PEG-lipid may be 10 to 40:10 to 70:1 to 70:1 to 5. In addition, the molar ratio may be 15 to 35:15 to 65:5 to 65:1 to 4 or 20 to 30:20 to 60:10 to 60:1 to 3, but the disclosure is not limited thereto.

A lipid nanoparticle including the DOTAP may further include an anionic drug, and the lipid nanoparticle may have a diameter of, for example, 30 nm to 100 nm, specifically, 40 nm to 100 nm, and more specifically, 50 nm to 90 nm, but the disclosure is not limited thereto.

## Drug delivery and pharmaceutical composition including lipid nanoparticle

Another aspect of the disclosure relates to a drug delivery composition including an anionic drug-containing lipid nanoparticle according to the disclosure.

Another aspect of the disclosure relates to a pharmaceutical composition including an anionic drug-containing lipid nanoparticle according to the disclosure as an active ingredient.

The lipid nanoparticle and the anionic drug are each as described above.

The lipid nanoparticle of the disclosure forms a stable complex with anionic drugs such as nucleic acids and exhibits low cytotoxicity and effective cell absorption, and thus is effective in delivering anionic drugs. Therefore, the lipid nanoparticle has a preventive or therapeutic effect on related diseases according to the type of anionic drug used and the type of nucleic acid, and has unlimited potential for use as a drug delivery composition. In addition, the lipid nanoparticle may be delivered specifically to the liver or lungs upon administration, and thus may target desired tissues depending on the purpose.

In the disclosure, the term "therapeutic" refers to intervention for altering the natural process of an individual or cell with a disease, which may be carried out either during the progression of pathological conditions or as a preventive measure. Desired therapeutic effects include preventing the onset or recurrence of disease, alleviating symptoms, mitigating all direct or indirect pathological consequences of the disease, preventing progression, slowing the rate of disease progression, alleviating or palliating the disease status, and achieving remission or improving prognosis. In particular, in the disclosure, they include all actions that improve the course of a disease by administering lipid nanoparticles including a sulfide-containing ionizable lipid, a stereoisomer thereof, or a pharmaceutically acceptable salt thereof, and an anionic drug as an active ingredient. In addition, the term "preventive" refers to all actions that suppress or delay the onset of a disease by administering the lipid nanoparticles. When the lipid nanoparticles of the disclosure are used for therapeutic or preventive purposes, they are administered to a subject in a therapeutically effective amount.

The term "therapeutically effective amount" used in the disclosure refers to an effective amount of anionic drug-containing lipid nanoparticles. Specifically, "therapeutically effective amount" refers to an amount sufficient to treat a

disease at a reasonable benefit/risk ratio applicable to medical treatment, and the effective dosage level may be determined based on factors such as the type and severity of entity, age, gender, type of disease, drug activity, sensitivity to drugs, time of administration, route of administration, and excretion rate, duration of treatment, concurrently used drugs, and other factors well known in the medical field. The pharmaceutical composition of the disclosure may be administered as an individual therapeutic agent or in combination with other therapeutic agents, or may be administered sequentially or simultaneously with commercially available therapeutic agents. And the pharmaceutical composition may be administered in a single dose or multiple doses. Taking all these factors into consideration, it is important to administer the minimum amount that allows maximum effects to be achieved without causing side effects, which may be easily determined by those skilled in the art. The administration dosage of the pharmaceutical composition of the disclosure may be determined by experts based on various factors such as a patient's condition, age, gender, and complication. The active ingredient of the composition of the disclosure is highly safe and thus may be used in doses greater than or equal to the determined administration dosage.

A composition including the lipid nanoparticle as an active ingredient may be administered orally, intramuscularly, intravenously, arterially, subcutaneously, intraperitoneally, via pulmonary route, and nasally, but the disclosure is not limited thereto.

The composition of the disclosure may further include at least one pharmaceutically acceptable carrier for administration. A pharmaceutically acceptable carrier may include saline, sterile water, Ringer's solution, buffered saline, dextrose solution, maltodextrin solution, glycerol, ethanol, and a mixture of one or more thereof, and as necessary, other common additives such as antioxidants, buffers, and bacteriostatic agents may be added. In addition, diluents, dispersants, surfactants, binders, and lubricants may be further added to formulate injectable formulations such as aqueous solutions, suspensions, emulsions, etc., pills, capsules, granules, or tablets. Therefore, the composition of the disclosure may be in the form of patches, pills, capsules, granules, tablets, suppositories, etc. These preparations may be prepared using methods commonly employed in formulation in the art or using methods disclosed in the literature [Remington's Pharmaceutical Science, Mack Publishing Company, Easton PA], and may be formulated into various preparations tailored to each disease or ingredient.

The embodiments of the disclosure may be modified into various other forms, and the scope of the disclosure is not limited to the embodiments described below. In addition, the embodiments of the disclosure are provided to more completely explain the disclosure to those with average knowledge in the relevant technical field. Furthermore, throughout the specification, the expression "including" an element means that another element may be further included, rather than excluding the existence of the other element, unless otherwise described.

**[Mode for Invention]**

Hereinafter, the configuration and effects of the disclosure will be described in detail through examples. The following examples are only for illustrating the disclosure, and the scope of the disclosure is not limited by the following examples.

**Preparation Example. Preparation of sulfide-containing ionizable lipid**

[Reaction Scheme 1]

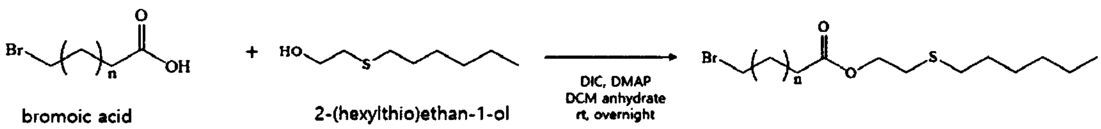


bromoic acid

2-(hexylthio)ethan-1-ol

First, using the synthetic method of Reaction Scheme 1, bromic acid and 2-(hexylthio)ethan-1-ol were reacted in a molar ratio of 1:1 to produce a sulfide-containing tail group having various alkyl chain lengths.

[Reaction Scheme 2]

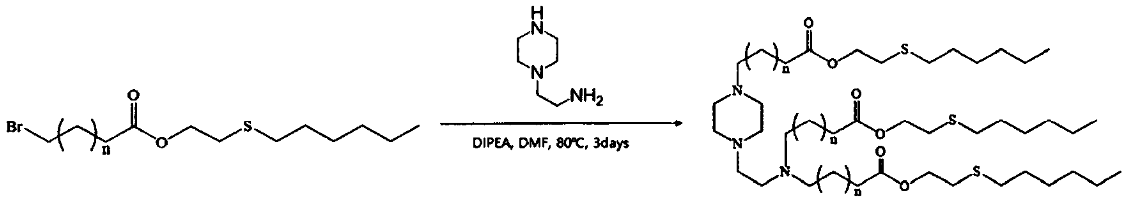


Next, using the synthetic method of Reaction Scheme 2, the tail group and N-(2-aminoethyl)piperazine as a head group were reacted in a molar ratio of 2.36:0.7 to synthesize a sulfide-containing ionizable lipid having a linear structure, named 244nHHES (wherein n is the number of carbon atoms from the head group to the ester group).

### Example 1-1: Preparation of Compounds 1 to 4

Detailed examples of the sulfide-containing ionizable lipid of the disclosure are shown in Table 2 below.

[Table 2]

| Number | Final product (Ionizable lipid) |
|---|---|
| Compound 1 (2446HHES) | 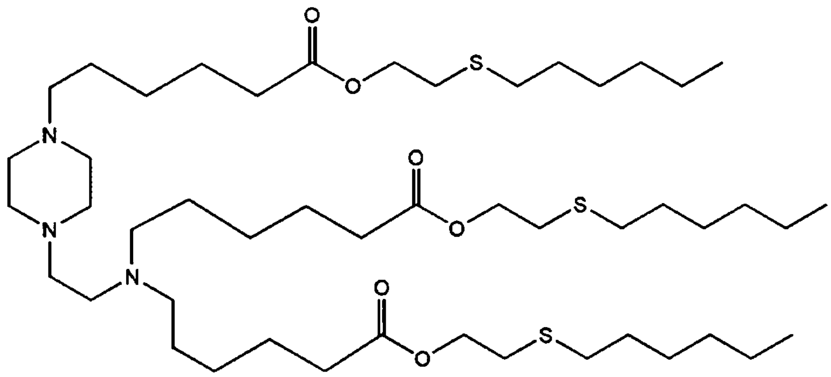 |
| Compound 2 (2447HHES) | 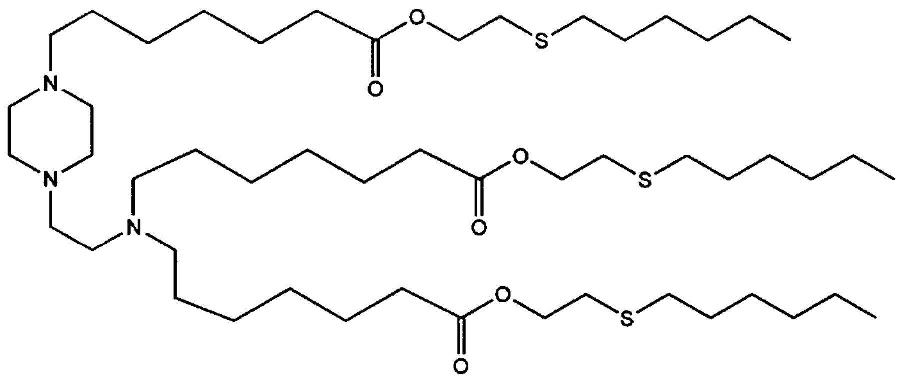 |
| Compound 3 (2448HHES) | 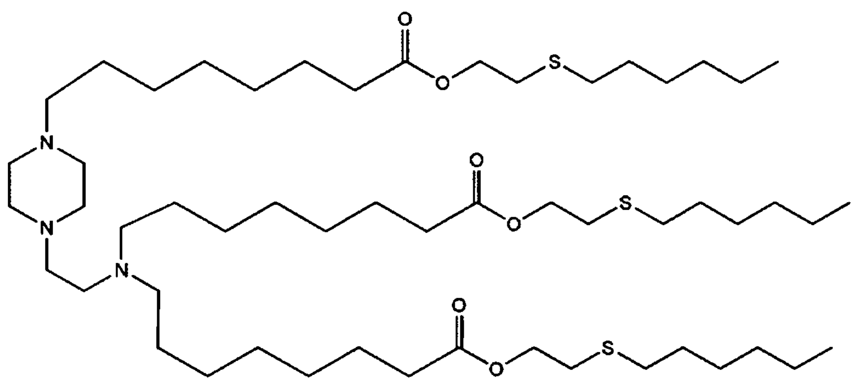 |

(continued)

| Number | Final product (Ionizable lipid) |
|---|---|
| Compound 4 (2449HHES) | 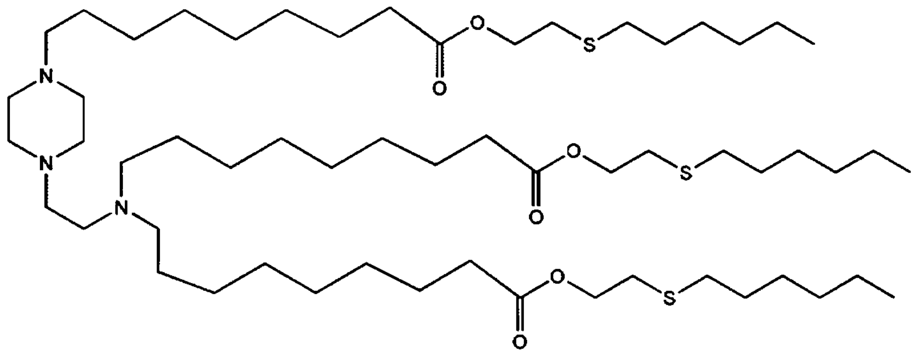 |

As an example, a detailed preparation method of Compound 3 (2448HHES) is as follows. 3 mmol of 2-(hexylthio) ethan-1-ol and 3 mmol of 8-bromooctanoic acid were added to 1 ml of dichloromethane (DCM) solvent, and to this mixture, 4.6 mmol of N-(3-dimethylaminopropyl)-N'-ethylcarbodiimide hydrochloride containing N,N-diisopropylcarbodiimide (DIC) and 0.6 mmol of 4-dimethylaminopyridine (DMAP) were added, followed by stirring for 18 hours at room temperature. Next, the reaction product was filtered and evaporated under vacuum. The residue was purified by column chromatography using gradient elution from 100 % hexane to a 95:5 volume ratio of hexane:ethyl acetate, using a CombiFlash RF system with a RediSep Gold Resolution silica column (Teledyne Isco). As a result, 2.36 mmol of 2-(hexylthio)ethyl 8-bromooctanoate was synthesized with a yield of 76.6 % and was named 8HHES.
Next, 2.36 mmol of 2-(hexylthio)ethyl 8-bromooctanoate, 0.7 mmol of N-(2-aminoethyl)piperazine, and 0.7 mmol of N,N-diisopropylethylamine were added to 1 ml of N,N-dimethylformamide (DMF) solvent, followed by stirring for three days at 80°C and then evaporation under vacuum. The residue was purified by column chromatography using gradient elution from 100 % DCM to a 95:4.5:0.5 volume ratio of DCM:MeOH:$NH_4OH$, using a CombiFlash RF system with a RediSep Gold Resolution silica column (Teledyne Isco). As a result, the ionizable lipid was produced with a yield of 36.6 %.
The remaining example compounds were prepared in a similar manner to Reaction Scheme 1 and Reaction Scheme 2.

**Example 1-2. Confirmation of synthesis of sulfide-containing ionizable lipid**

In order to confirm the synthesis of Compounds 1 to 4 produced in Example 1-1, MS analysis was conducted. The results are shown in Table 3 below.

[Table 3]

| Ionizable lipid | Chemical Formula | Observed m/z ratio |
|---|---|---|
| Compound 1 (2446HHES) | $C_{48}H_{93}N_3O_6S_3$ | 904.6306 $[M+H]^+$ |
| Compound 2 (2447HHES) | $C_{51}H_{99}N_3O_6S_3$ | 946.6769 $[M+H]^+$ |
| Compound 3 (2448HHES) | $C_{54}H_{105}N_3O_6S_3$ | 988.7240 $[M+H]^+$ |
| Compound 4 (2449HHES) | $C_{57}H_{111}N_3O_6S_3$ | 1030.7707 $[M+H]^+$ |

**Example 2. Preparation of nucleic acid-encapsulating lipid nanoparticles**

Example 2-1. Preparation of helper lipid (DOPE)-containing lipid nanoparticles

The produced ionizable lipids (2446HHES to 2449HHES), cholesterol (Cholesterol powder, BioReagent, suitable for cell culture, ≥99 %, Sigma, Korea), DOPE (18:1 (△9-Cis) PE (DOPE), Avanti , USA), and C16-PEG2000 Ceramide (C16 PEG2000 Ceramide, Avanti, USA) were dissolved in ethanol in a molar ratio of 26.5:52:20:1.5. In addition, 30 μg of mRNA as an RNA therapeutic agent was diluted in 0.75 ml of sodium citrate.
An organic phase (ethanol) in which ionizable lipids, cholesterol, helper lipids, and lipid-PEG were dissolved and an aqueous phase (sodium acetate or sodium citrate) in which mRNA was dissolved were mixed through a microfluidic mixing device (Benchtop Nanoassemblr; PNI, Canada) at a flow rate of 12 ml/min to produce nucleic acid-encapsulating

lipid nanoparticles. In order to produce luciferase mRNA (SEQ ID NO: 1)-encapsulating lipid nanoparticles, an organic phase and an aqueous phase were mixed so that a weight ratio of mRNA:ionizable lipid was 1:10, to produce lipid nanoparticles. Afterwards, in order to remove ethanol and match the pH of the body and the pH of lipid nanoparticles to each other, dialysis was conducted with phosphate buffered saline (PBS) for 16 hours using a 3500 MWCO dialysis cassette.

Example 2-2. Preparation of helper lipid (DOTAP)-containing lipid nanoparticles

The ionizable lipid (2448HHES):DOTAP (1,2-dioleoyl-3-trimethylammonium, Avanti, USA):cholesterol:lipid-PEG (C16-PEG2000 Ceramide) were dissolved in ethanol in a molar ratio of 26.5:20 to 60:12 to 52:1.5, and an organic phase and an aqueous phase were mixed so that a weight ratio of mRNA:ionizable lipid was 1:10, to produce lipid nanoparticles (Table 4). In order to remove ethanol and match the pH of the body and the pH of lipid nanoparticles, dialysis was conducted with PBS for 16 hours using a 3500 MWCO dialysis cassette.

[Table 4]

| | 0 % DOTAP | 20 % DOTAP | 40 % DOTAP | 60 % DOTAP |
|---|---|---|---|---|
| Ionizable lipid (2448HHES) | 26.5 | 26.5 | 26.5 | 26.5 |
| DOTAP (DOPE) | 0 (20) | 20 (0) | 40 (0) | 60 (0) |
| Cholesterol | 52 | 52 | 32 | 12 |
| C16 PEG | 1.5 | 1.5 | 1.5 | 1.5 |

**Experimental Example 1. Confirmation of physical and chemical properties of lipid nanoparticles**

Experimental Example 1-1. Measurement of drug encapsulation rate

The drug encapsulation rate (encapsulation efficiency, %) of each lipid nanoparticle encapsulating mRNA as a nucleic acid drug was measured through Ribogreen analysis (Quant-iT™ RiboGreen® RNA, Invitrogen). The lipid nanoparticles were diluted with 50 μl of 1xTE buffer so that the final concentration of mRNA was 4 μg/ml to 7 μg/ml in a 96-well plate. For groups not treated with Triton-X (Triton-X LNP(-)), 50 μl of 1xTE buffer was added, and for groups treated with Triton-X (Triton-X LNP(+)), 50 μl of 2 % Triton-X buffer was added. After incubating at 37°C for 10 minutes, the lipid nanoparticles were broken down using Triton-X to release encapsulated nucleic acids. Afterwards, 100 μl of Ribogreen reagent was added to each well. The fluorescence intensity (FL) of Triton LNP(-) and Triton LNP(+) was measured using Infinite® 200 PRO NanoQuant (Tecan) with wavelength bandwidth (excitation: 485 nm, emission: 528 nm), and the drug encapsulation rate (encapsulation efficiency, %) was calculated according to Equation 1 below.

Drug encapsulation rate (%) = (Fluorescence of Triton LNP(+) - Fluorescence of Triton LNP(-)) / (Fluorescence of Triton LNP(+)) X 100 [Equation 1]

[Table 5]

| Lipid nanoparticle | Drug encapsulation rate (Encapsulation efficiency, %) |
|---|---|
| 2447HHES | 89.6 |
| 2448HHES | 92.7 |
| 2449HHES | 84.3 |

As confirmed in Table 5, it was confirmed that the lipid nanoparticles according to the disclosure could encapsulate drugs with high efficiency.

**Experimental Example 1-2. Measurement of particle size**

The sizes of the produced nucleic acid-encapsulating lipid nanoparticles were measured. RNA (luciferase mRNA) included in each lipid nanoparticle produced in Example 2 was diluted using PBS to a concentration of 1 μg/ml, and

the diameters and polydispersity indices (PDIs) of the LNPs were measured using dynamic light scattering (DLS) with a Malvern Zetasizer Nano (Malvern Instruments, UK).

The respective result values are as follows (Table 6).

[Table 6]

| LNP | Diameter (nm) | PDI |
|---|---|---|
| 2447HHES | 69.58 | 0.249 |
| 2448HHES | 60.40 | 0.192 |
| 2449HHES | 49.26 | 0.247 |

**Experimental Example 2. Confirmation of in vitro efficacy of nucleic acid-encapsulating lipid nanoparticles**

In order to confirm the in vitro efficacy of nucleic acid-encapsulating lipid nanoparticles, screening was conducted using lipid nanoparticles synthesized with various tail groups.

The test was conducted by delivering luciferase mRNA to HeLa, HepG2, HEK 293, and MRC-5 cells using lipid nanoparticles (Compounds 1 to 4), each synthesized with various tail groups, followed by measuring luminescence intensity to confirm expression of the gene.

Specifically, one day before transfecting the cells with the lipid nanoparticles, HeLa, HepG2, HEK 293, and MRC-5 cells (from the Korean Cell Line Bank) were dispended into a white (96-well) plate at a density of 0.01 x $10^6$ cells/well and cultured in DMEM media (SH30022, Hyclone, USA) at 37°C with 0.5-3 % $CO_2$. The cells were treated with luciferase mRNA-encapsulating lipid nanoparticles at a dose of 25 ng/well based on mRNA included in the lipid nanoparticles. After 24 hours, Bright-Glo™ luciferase assay solution (promega, USA) was treated at 100 μl/well and then left at room temperature for 10 minutes. The luminescence intensity of the dissolved cells was then measured using an Infinite M200 luminescence meter (Tecan, USA).

As confirmed in FIG. 1, it was confirmed that the luminescence intensity was significantly increased in all lipid nanoparticles, compared to SM-102 as a positive control group.

**Experimental Example 3. Confirmation of in vivo efficacy of lipid nanoparticles**

Experimental Example 3-1. Effects of luciferase mRNA-encapsulating lipid nanoparticles

Luciferase mRNA-encapsulating lipid nanoparticles were produced, and each nanoparticle was dialyzed in PBS for 16 hours to remove ethanol. mRNA-encapsulating lipid nanoparticles were injected intravenously or intramuscularly at a dose of 0.1 mg/kg based on mRNA included in lipid nanoparticles into a C57BL/6 female 7-week-old mouse (Orient bio), and then, after six hours, 0.25 mg/kg of luciferin was administered intraperitoneally, to confirm bioluminescence using IVIS (PerkinElmer, USA) equipment. The mouse administered with luciferase mRNA-encapsulating lipid nanoparticles was sacrificed, its organs were harvested, and the biodistribution of lipid particles in each organ was confirmed using IVIS equipment.

As shown in FIGS. 2 and 3, as a result of the intravenous injection, it was confirmed that lipid nanoparticles encapsulating luciferase mRNA and including HHES ionizable lipids exhibited a high luminescence intensity specifically in the liver through whole-body imaging and ex vivo organ imaging.

In addition, as a result of the intramuscular injection, it was confirmed that lipid nanoparticles encapsulating luciferase mRNA and including HHES ionizable lipids emitted light at the injection site. (FIGS. 4 and 5)

**Experimental Example 3-2. Effects of hEPO mRNA-encapsulating lipid nanoparticles**

Lipid nanoparticles loaded with hEPO mRNA (SEQ ID NO: 2) were administered to a mouse through intravenous injection at an mRNA dose of 0.5 mg/kg. Blood was collected six hours after the injection, and serum was separated from the blood. hEPO and MCP-1 levels were confirmed using an hEPO ELISA kit (DEP00; R&D systems, Minneapolis, MN, USA) and an MCP-1 ELISA kit (BMS281; Thermo Fisher, Waltham, MA, USA), respectively.

As a result, as shown in FIG. 6, it was confirmed that ionizable lipid-containing lipid nanoparticles of the disclosure exhibited a significantly higher level of EPO protein in the blood compared to MC-3 and a significantly lower MCP-1 level compared to SM-102. MCP-1 is an indicator of the initial immunogenicity of lipid nanoparticles, and it was confirmed that ionizable lipid-containing lipid nanoparticles of the disclosure had a significantly lower initial immunogenicity compared to SM-102.

**Experimental Example 4. Confirmation of properties and effects of helper lipid (DOTAP)-containing lipid nanoparticles**

Experimental Example 4-1. Confirmation of properties of lipid nanoparticles according to proportion of DOTAP included

The physical and chemical properties of the lipid nanoparticles produced in Example 2-2 were confirmed and are shown in Table 7 below. Even when DOTAP was used as a helper lipid, an excellent drug encapsulation rate was confirmed.

[Table 7]

| | EE (%) | Diameter (nm) | PDI | Zeta (mV) |
|---|---|---|---|---|
| 0 % DOTAP | 94.5 | 75.4 ± 12.5 | 0.092 | -0.96 ± 0.15 |
| 20 % DOTAP | 98.8 | 53.7 ± 1.5 | 0.139 | -2.82 ± 0.15 |
| 40 % DOTAP | 99.0 | 61.1 ± 3.3 | 0.142 | 1.51 ± 0.12 |
| 60 % DOTAP | 98.8 | 59.35 ± 6.3 | 0.131 | 4.70 ± 0.35 |

**Experimental Example 4-2. Confirmation of in vivo efficacy according to proportion of DOTAP included**

Luciferase mRNA-encapsulating lipid nanoparticles were injected intravenously at a dose of 0.1 mg/kg based on mRNA included in lipid nanoparticles into a C57BL/6 female 7-week-old mouse (Orient bio), and then, after six hours, 0.25 mg/kg of luciferin was administered intraperitoneally, to confirm bioluminescence through IVIS (PerkinElmer, USA) equipment. The mouse administered with luciferase mRNA-encapsulating lipid nanoparticles was sacrificed, its organs were harvested, and the biodistribution of lipid particles in each organ was confirmed through IVIS equipment.

As a result, it was confirmed that luciferase mRNA-encapsulating lipid nanoparticles exhibited a high luminescence intensity in the lungs through whole-body imaging and ex vivo organ imaging (FIG. 7). Therefore, it was confirmed that lipid nanoparticles including ionizable lipids and DOTAP of the disclosure delivered nucleic acids specifically to the lungs.

From the above description, those skilled in the art to which the disclosure belongs will be able to understand that the disclosure can be implemented in other specific forms without changing its technical idea or essential features. In this regard, it should be understood that the embodiments described above are illustrative in all respects and not restrictive. The scope of the disclosure should be interpreted to include all changes or modifications derived from the meaning and scope of the claims and their equivalents, rather than from the detailed description above.

## Claims

1. An ionizable lipid represented by Chemical Formula 1, a stereoisomer thereof, or a pharmaceutically acceptable salt thereof:

[Chemical Formula 1]

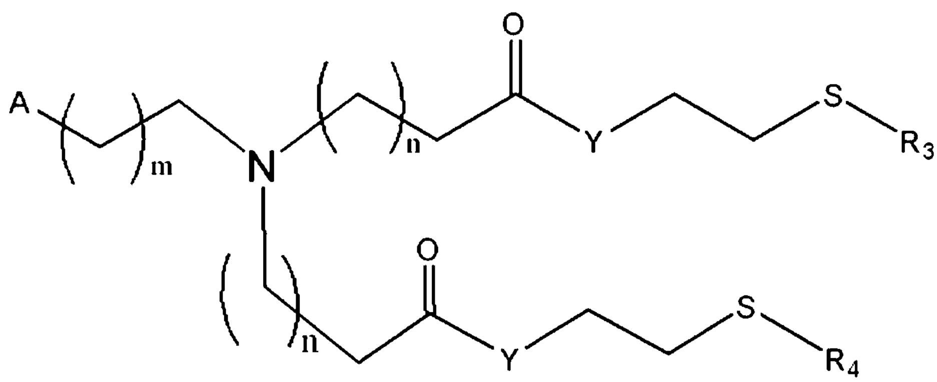

wherein, A is

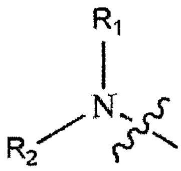

or

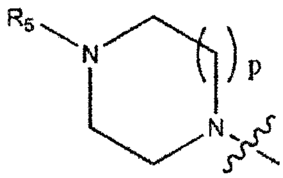

,

$R_1$ and $R_2$ are each independently any one selected from -H, -$C_{1-6}$alkyl , -$C_{1-6}$alkyl-$NR_3R_4$, and -$C_{2-12}$alkyl-(C=O)-Y-$(CH_2)_x$S-$C_{4-12}$alkyl,
$R_3$ and $R_4$ are each independently $C_{2-8}$alkyl,
$R_5$ is -$C_{2-12}$alkyl-(C=O)-Y-$(CH_2)_x$S-$C_{4-12}$alkyl,
Y is -O- or $NR_6$,
$R_6$ is -H or -$C_{1-3}$alkyl,
m is an integer from 0 to 3,
n is independently an integer from 2 to 7,
x is an integer from 1 to 3, and
p is an integer from 0 to 2.

**2.** The ionizable lipid, the stereoisomer thereof, or the pharmaceutically acceptable salt thereof, of claim 1, wherein

A is

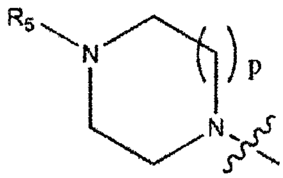

,

$R_5$ is -$C_{2-12}$alkyl-(C=O)-Y-$(CH_2)_x$S-$C_{4-12}$alkyl,
Y is -O-,
m is an integer from 0 to 3,
n is independently an integer from 2 to 7,
x is an integer from 1 to 3, and
p is 1.

**3.** The ionizable lipid, the stereoisomer thereof, or the pharmaceutically acceptable salt thereof, of claim 1, wherein the ionizable lipid is selected from the group consisting of Compounds 1 to 4 shown in a table below.

| Compound | Structure |
|---|---|
| 1 | 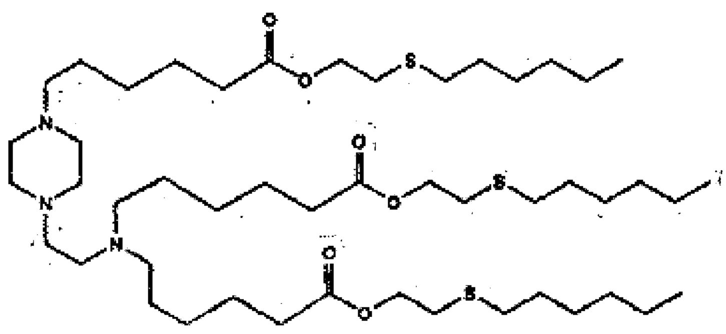 |

(continued)

| Compound | Structure |
|---|---|
| 2 | 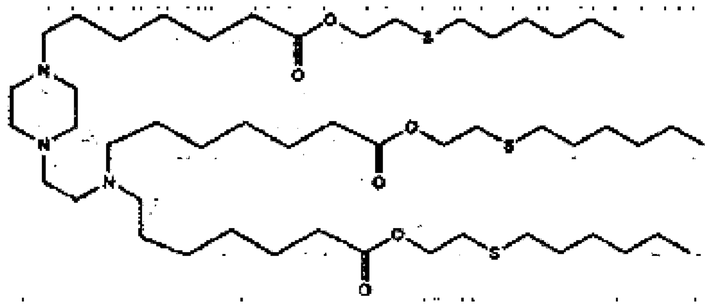 |
| 3 | 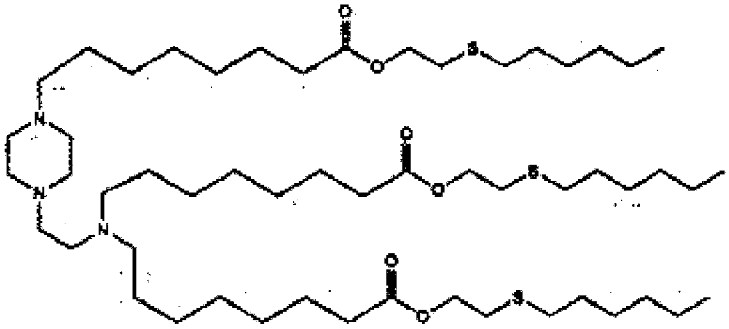 |
| 4 | 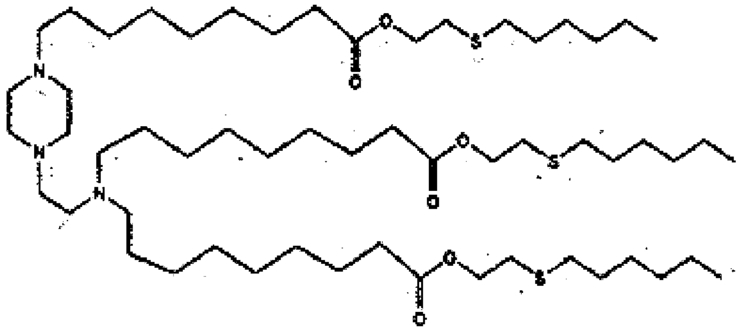 |

4. A lipid nanoparticle comprising the ionizable lipid, the stereoisomer thereof, or the pharmaceutically acceptable salt thereof, according to any one of claims 1 to 3.

5. The lipid nanoparticle of claim 4, further comprising at least any one selected from the group consisting of a helper lipid, a structural lipid, and a PEG-lipid.

6. The lipid nanoparticle of claim 5, wherein the helper lipid is at least any one selected from the group consisting of DOPE, DSPC, POPC, EPC, DOPC, DPPC, DOPG, DPPG, DSPE, DOTAP, phosphatidylethanolamine, dipalmitoylphosphatidylethanolamine, 1,2-dioleoyl-sn-glycero-3-phosphate, 1,2-dilinoleoyl-sn-glycero-3-phosphocholine, 1,2-diarachidonoyl-sn-glycero-3-phosphocholine, 1,2-didocosahexaenoyl- sn-glycero-3-phosphocholine, 1,2-diphytanoyl-sn-glycero-3-phosphoethanolamine, POPE, DOPS, DLPC, DMPC, DUPC, 1,2-di-O-octadecenyl-sn-glycero-3-phosphocholine, 1-oleoyl-2-cholesterylhemisuccinoyl-sn-glycero-3-phosphocholine, 1-hexadecyl-sn-glycero-3-phosphocholine, 1,2-distearoyl-sn-glycero-3-phosphoethanolamine, 1,2-dilinoleoyl-sn-glycero-3-phosphoethanolamine, 1,2-dilinoleoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, and sphingomyelin.

7. The lipid nanoparticle of claim 5, wherein the PEG-lipid is at least any one selected from the group consisting of PEG-ceramide, PEG-DMG, PEG-c-DOMG, PEG-DLPE, PEG-DMPE, PEG-DPPC, and PEG-DSPE.

8. The lipid nanoparticle claim 5, wherein the structural lipid is at least any one selected from the group consisting of cholesterol, cholestenol, spinasterol, fecosterol, sitosterol, ergosterol, ergostenol, campesterol, stigmasterol, brassicasterol, tomatidine, ursolic acid, and alpha-tocopherol.

**9.** The lipid nanoparticle of claim 5, wherein the lipid nanoparticle comprises the ionizable lipid:the helper lipid:the structural lipid:the PEG-lipid in a molar ratio of 10 to 40:10 to 70:40 to 70:0.5 to 5.

**10.** The lipid nanoparticle of claim 5, further comprising an anionic drug.

**11.** The lipid nanoparticle of claim 10, wherein the anionic drug is at least any one selected from the group consisting of a nucleic acid, a low-molecular weight compound, a peptide, a protein, a protein-nucleic acid structure, and an anionic biopolymer-drug conjugate.

**12.** The lipid nanoparticle of claim 11, wherein the nucleic acid is at least any one selected from the group consisting of small interfering ribonucleic acid (siRNA), ribosomal ribonucleic acid (rRNA), deoxyribonucleic acid (DNA), complementary deoxyribonucleic acid (cDNA), aptamer, messenger ribonucleic acid (mRNA), transport ribonucleic acid (tRNA), sgRNA, antisense oligonucleotide, shRNA, miRNA, ribozyme, PNA, and DNAzyme.

**13.** The lipid nanoparticle of claim 12, wherein the total lipid/nucleic acid weight ratio in the lipid nanoparticle is 1 to 20.

**14.** The lipid nanoparticle of claim 10, wherein the lipid nanoparticle has an average diameter of 40 nm to 150 nm.

**15.** A drug delivery composition comprising the lipid nanoparticle according to claim 10.

**16.** The drug delivery composition of claim 15, wherein the drug delivery composition is delivered specifically to a liver.

**17.** The drug delivery composition of claim 15, wherein the lipid nanoparticle further comprises DOTAP and is delivered specifically to lungs.

**18.** The drug delivery composition of claim 17, wherein the DOTAP is included in the total lipid of the lipid nanoparticle at 10 mol% to 70 mol%.

FIG. 1

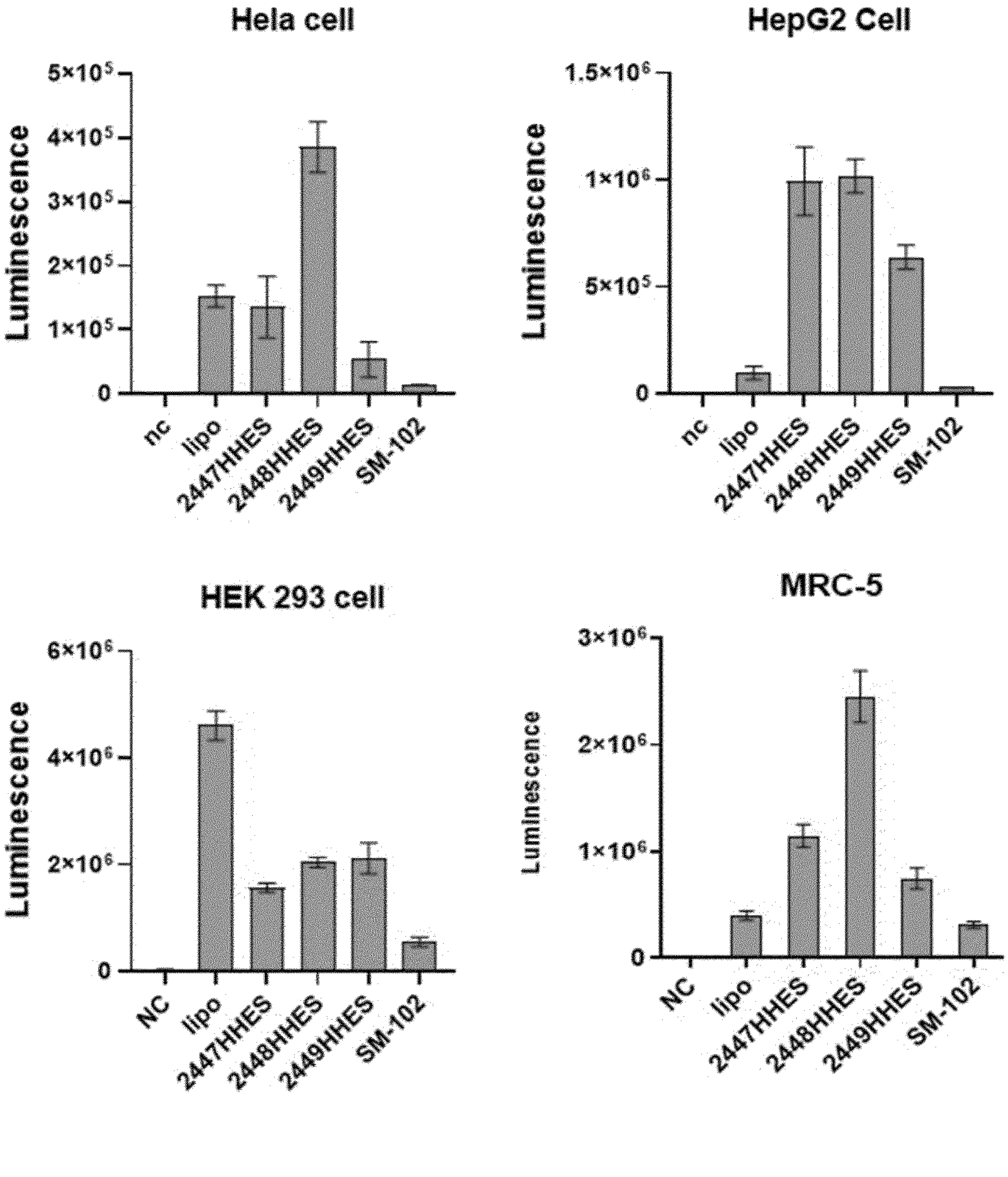
Hela cell
Luminescence
5×10^5
4×10^5
3×10^5
2×10^5
1×10^5
0
nc
lipo
2447HHES
2448HHES
2449HHES
SM-102
HepG2 Cell
Luminescence
1.5×10^6
1×10^6
5×10^5
0
nc
lipo
2447HHES
2448HHES
2449HHES
SM-102
HEK 293 cell
Luminescence
6×10^6
4×10^6
2×10^6
0
NC
lipo
2447HHES
2448HHES
2449HHES
SM-102
MRC-5
Luminescence
3×10^6
2×10^6
1×10^6
0
NC
lipo
2447HHES
2448HHES
2449HHES
SM-102

FIG. 2

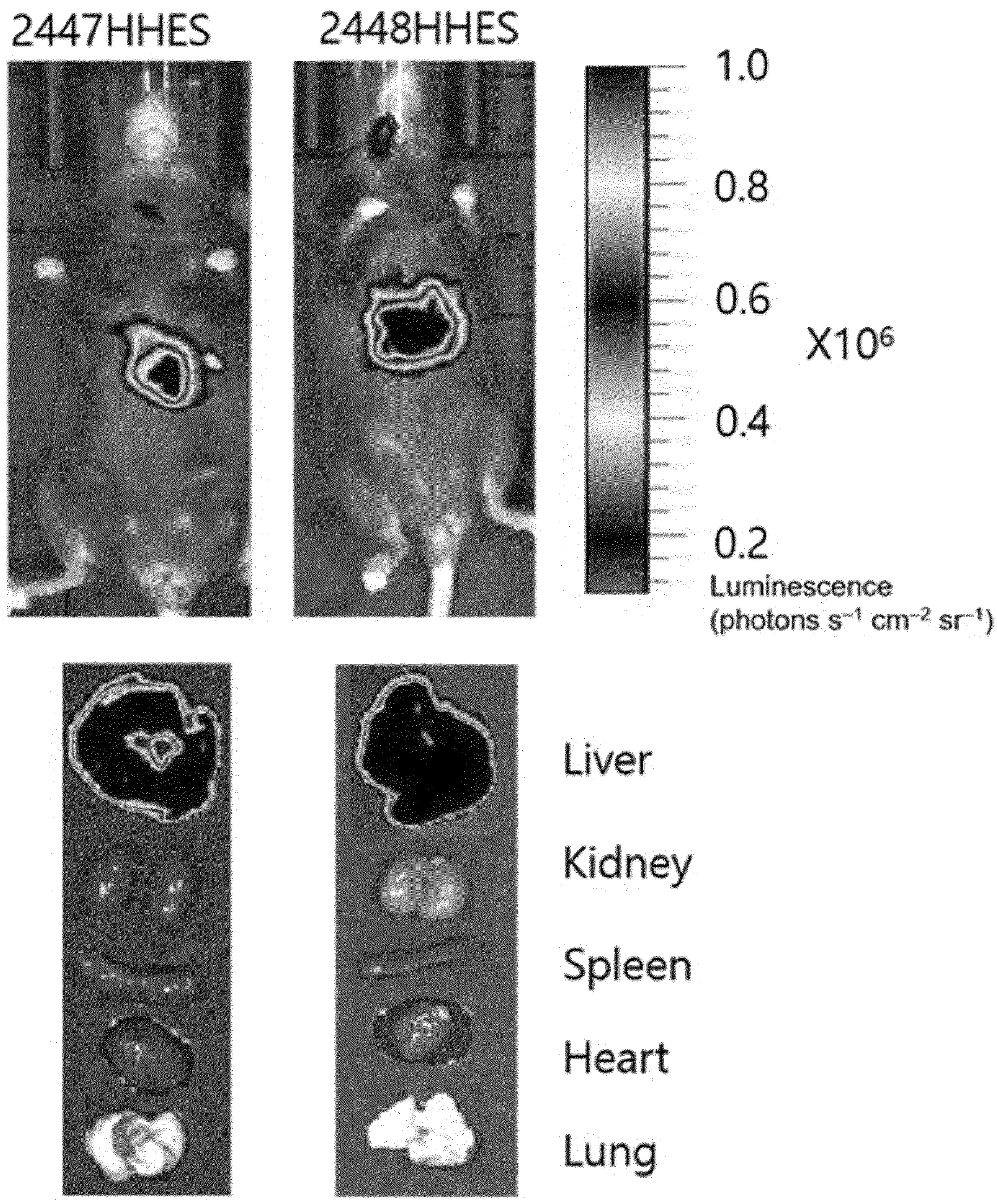
2447HHES
2448HHES
1.0
0.8
0.6
X10⁶
0.4
0.2
Luminescence
(photons s⁻¹ cm⁻² sr⁻¹)
Liver
Kidney
Spleen
Heart
Lung

FIG. 3

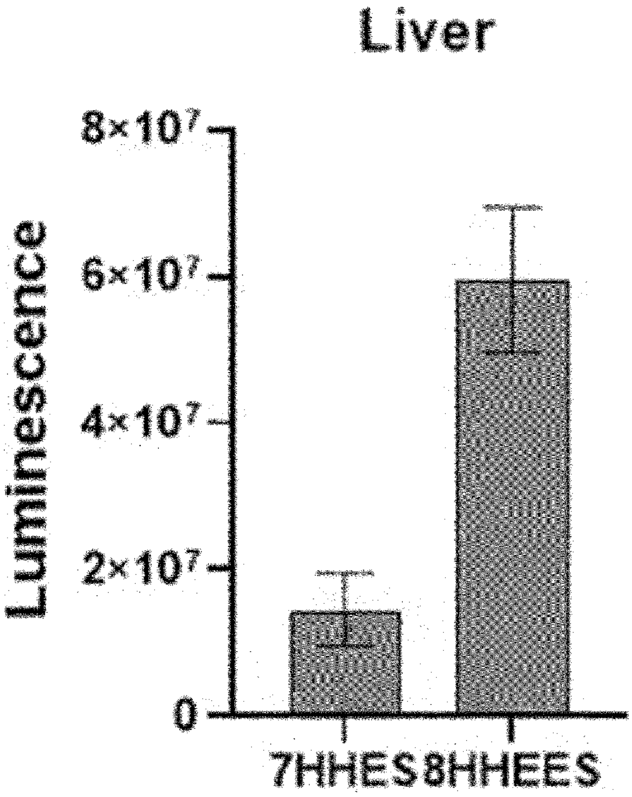

FIG. 4

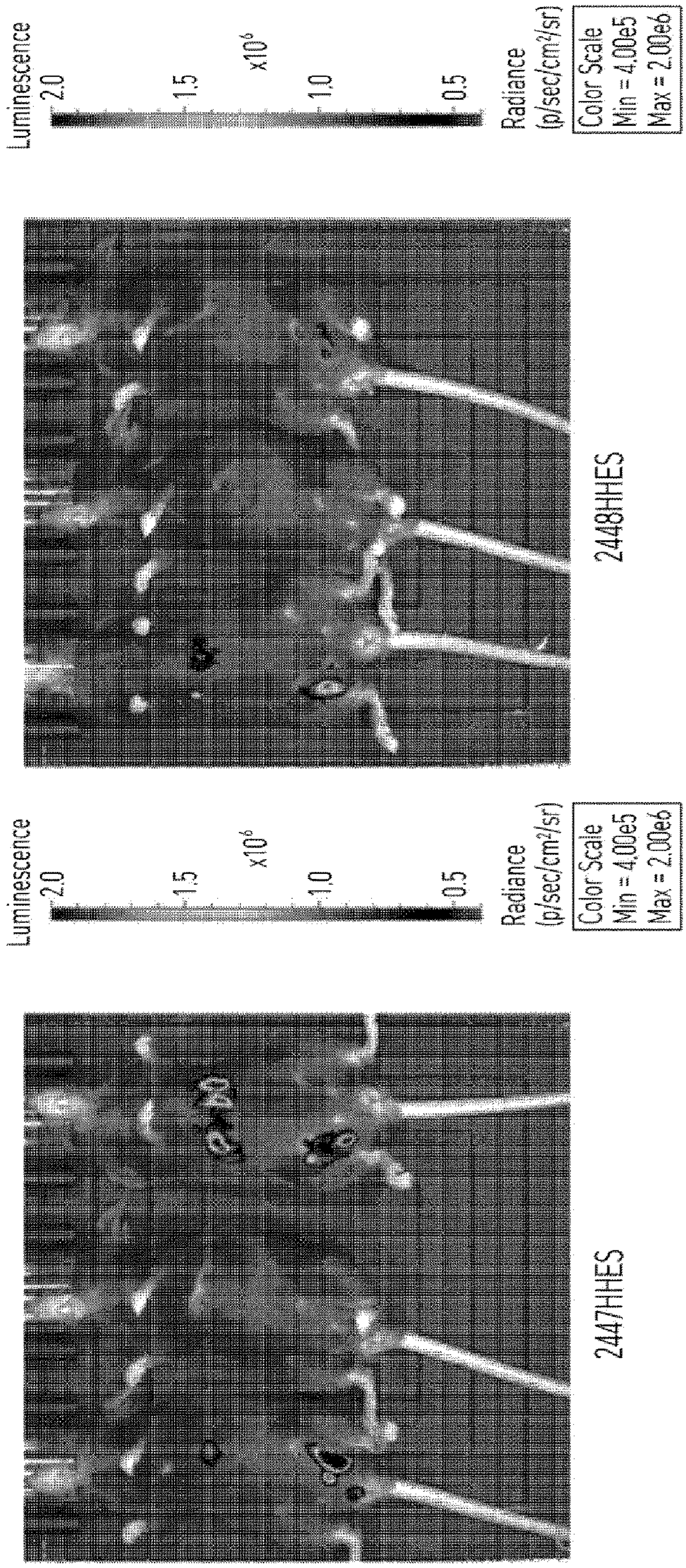

Luminescence
2.0
1.5
1.0
0.5
x10^6
Radiance
(p/sec/cm²/sr)
Color Scale
Min = 4.00e5
Max = 2.00e6
2448HHES
Luminescence
2.0
1.5
1.0
0.5
x10^6
Radiance
(p/sec/cm²/sr)
Color Scale
Min = 4.00e5
Max = 2.00e6
2447HHES

FIG. 5

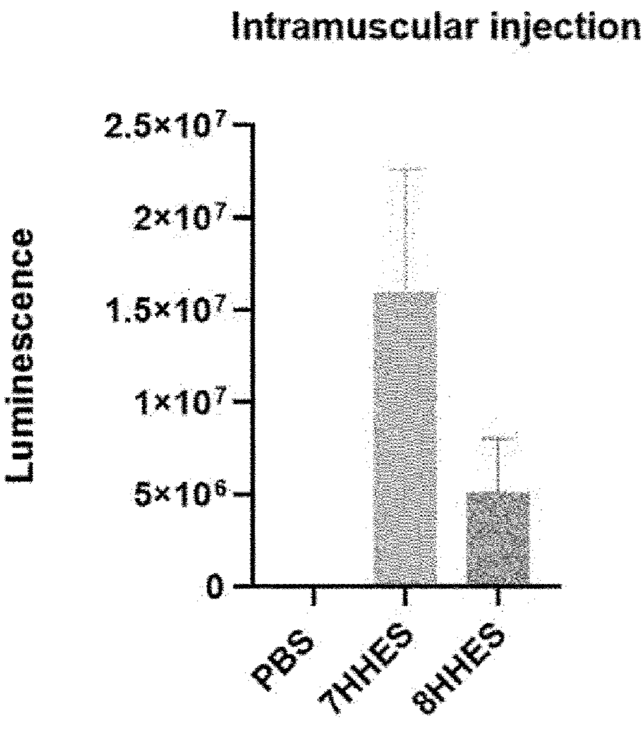
Intramuscular injection
Luminescence
2.5×10^7
2×10^7
1.5×10^7
1×10^7
5×10^6
0
PBS
7HHES
8HHES

FIG. 6

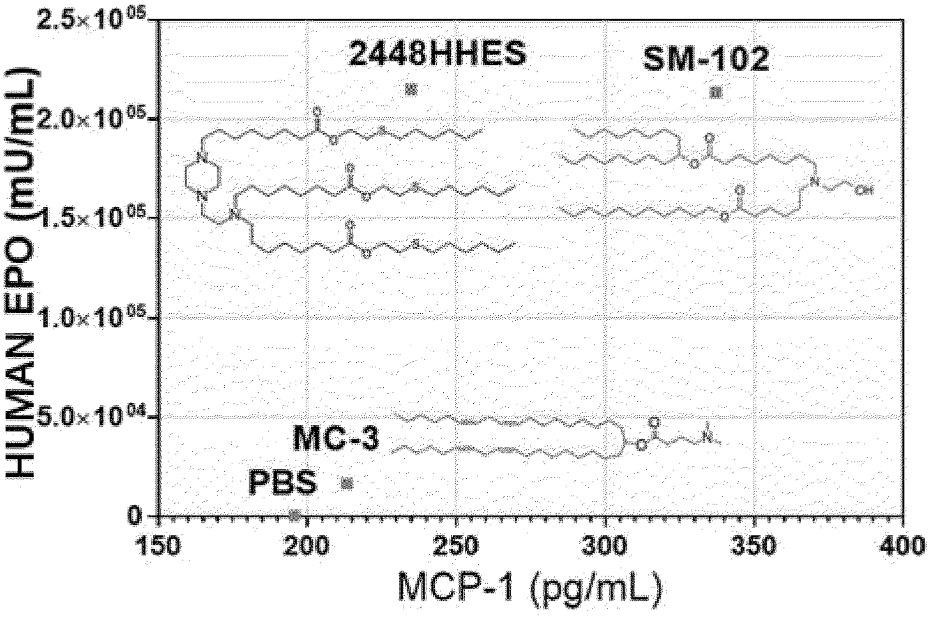
2448HHES
SM-102
MC-3
PBS
HUMAN EPO (mU/mL)
2.5×10^05
2.0×10^05
1.5×10^05
1.0×10^05
5.0×10^04
0
150
200
250
300
350
400
MCP-1 (pg/mL)

FIG. 7

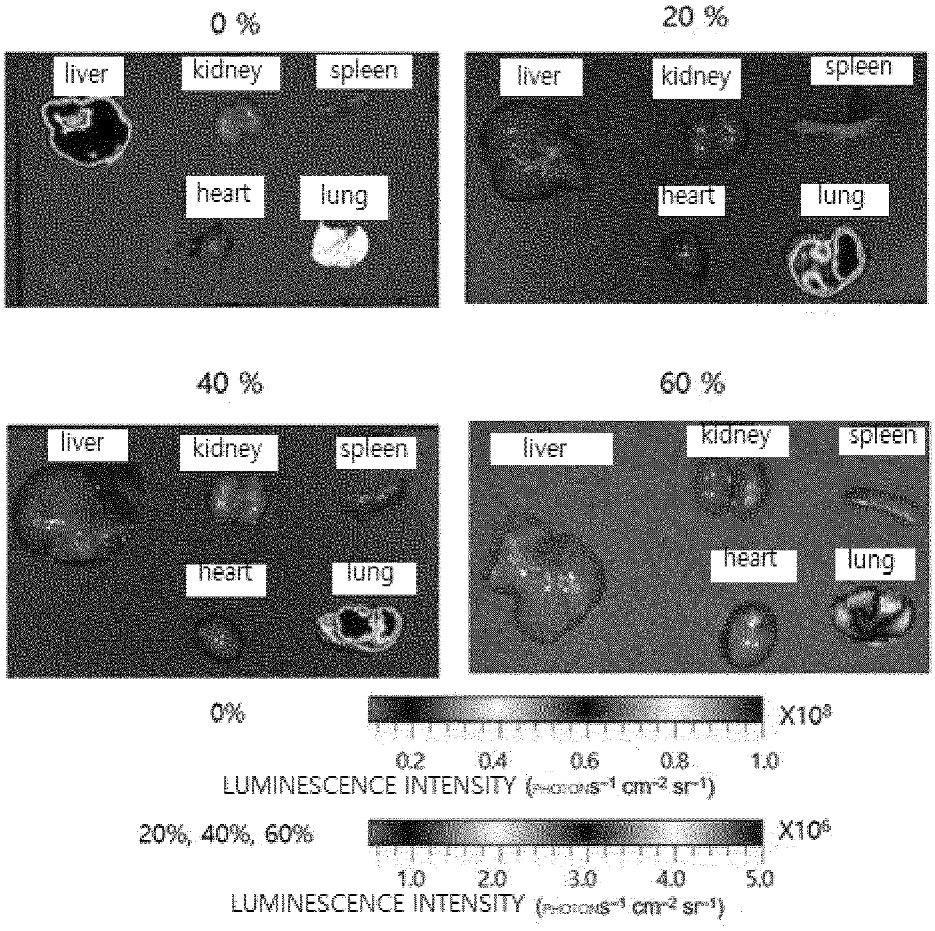
0 %
20 %
40 %
60 %
liver
kidney
spleen
heart
lung
0%
X10⁸
0.2 0.4 0.6 0.8 1.0
LUMINESCENCE INTENSITY (PHOTONS s-1 cm-2 sr-1)
20%, 40%, 60%
X10⁶
1.0 2.0 3.0 4.0 5.0
LUMINESCENCE INTENSITY (PHOTONS s-1 cm-2 sr-1)

FIG. 8

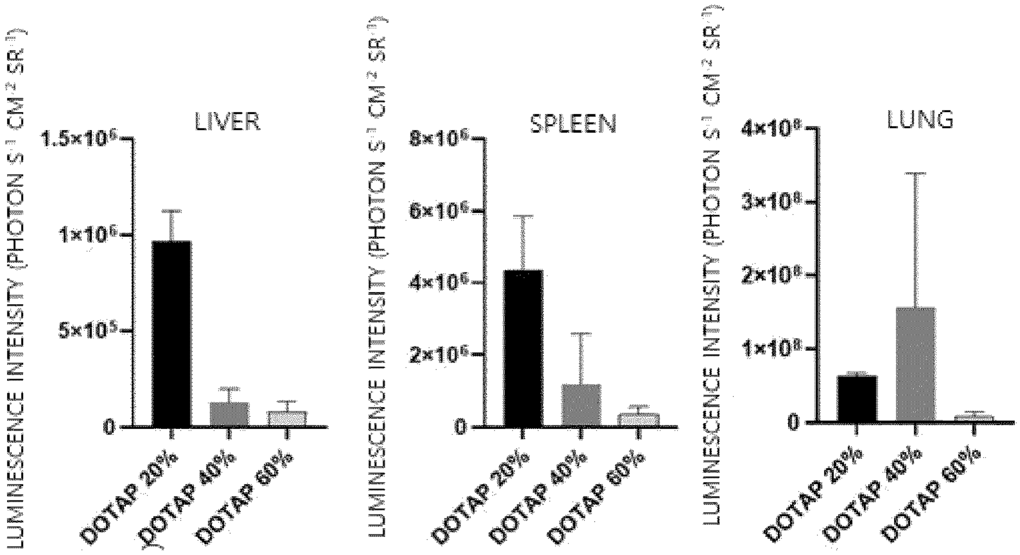
LIVER
SPLEEN
LUNG
LUMINESCENCE INTENSITY (PHOTON S-1 CM-2 SR-1)
1.5×10⁶
1×10⁶
5×10⁵
0
8×10⁶
6×10⁶
4×10⁶
2×10⁶
4×10⁸
3×10⁸
2×10⁸
1×10⁸
DOTAP 20%
DOTAP 40%
DOTAP 60%

FIG 9.

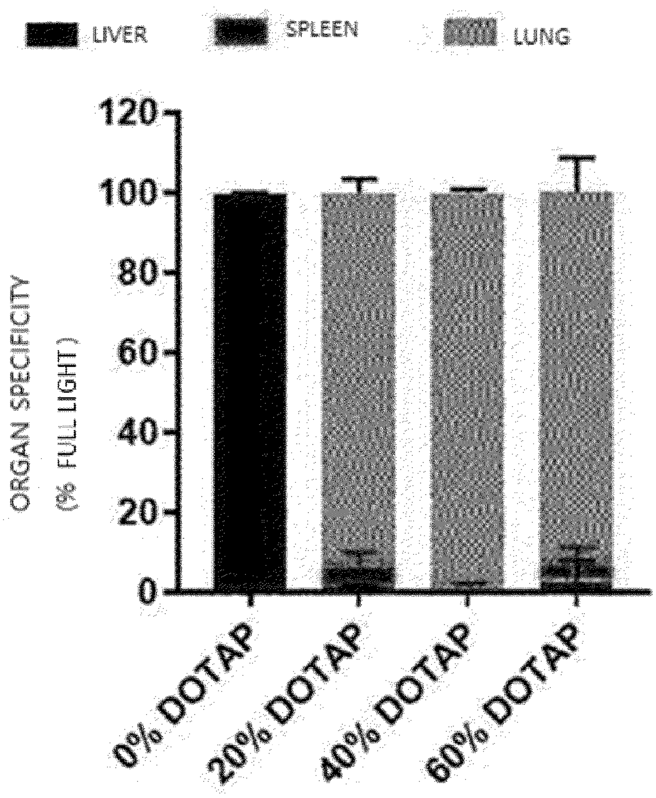
LIVER
SPLEEN
LUNG
ORGAN SPECIFICITY
(% FULL LIGHT)
120
100
80
60
40
20
0
0% DOTAP
20% DOTAP
40% DOTAP
60% DOTAP

**INTERNATIONAL SEARCH REPORT**

International application No.
**PCT/KR2024/000628**

**A. CLASSIFICATION OF SUBJECT MATTER**

**C07D 295/13**(2006.01)i; **A61K 9/51**(2006.01)i; **A61K 9/127**(2006.01)i; **A61K 47/22**(2006.01)i; **A61K 48/00**(2006.01)i

According to International Patent Classification (IPC) or to both national classification and IPC

**B. FIELDS SEARCHED**

Minimum documentation searched (classification system followed by classification symbols)

C07D 295/13(2006.01); A61K 47/48(2006.01); A61K 47/54(2017.01); A61K 47/69(2017.01); A61K 48/00(2006.01); A61K 9/16(2006.01); A61P 35/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 이온화 가능한 지질(ionizable lipid), 지질나노입자(lipid nanoparticle), 약물 전달(drug delivery)

**C. DOCUMENTS CONSIDERED TO BE RELEVANT**

| Category* | Citation of document, with indication, where appropriate, of the relevant passages | Relevant to claim No. |
|---|---|---|
| X | KR 10-2020-0115591 A (TRUSTEES OF TUFTS COLLEGE) 07 October 2020 (2020-10-07) See claims 1, 10 and 31; figure 1; and paragraphs [0006], [0009], [0010], [0030], [0075], [0076], [0094]-[0102], [0122], [0164], [0175] and [0213]. | 1,4-18 |
| A | | 2,3 |
| A | WO 2021-226092 A1 (TRUSTEES OF TUFTS COLLEGE) 11 November 2021 (2021-11-11) See entire document. | 1-18 |
| A | WO 2022-251665 A1 (RENAGADE THERAPEUTICS MANAGEMENT INC. et al.) 01 December 2022. See entire document. | 1-18 |
| A | WO 2017-212006 A1 (CUREVAC AG) 14 December 2017 (2017-12-14) See entire document. | 1-18 |
| A | WO 2014-186366 A1 (TUFTS UNIVERSITY) 20 November 2014 (2014-11-20) See entire document. | 1-18 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 April 2024** | **25 April 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/000628**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/KR2024/000628**

| Patent document cited in search report | Publication date (day/month/year) | Patent family member(s) | Publication date (day/month/year) |
|---|---|---|---|
| KR 10-2020-0115591 A | 07 October 2020 | CN 111954542 A | 17 November 2020 |
| | | EP 3746129 A1 | 09 December 2020 |
| | | EP 3746129 A4 | 02 March 2022 |
| | | JP 2021-512865 A | 20 May 2021 |
| | | US 2020-0368254 A1 | 26 November 2020 |
| | | US 2023-0405022 A1 | 21 December 2023 |
| | | WO 2019-152848 A1 | 08 August 2019 |
| WO 2021-226092 A1 | 11 November 2021 | CN 115867262 A | 28 March 2023 |
| | | EP 4146278 A1 | 15 March 2023 |
| | | JP 2023-524747 A | 13 June 2023 |
| | | KR 10-2023-0005367 A | 09 January 2023 |
| | | US 11666539 B2 | 06 June 2023 |
| | | US 2021-0346307 A1 | 11 November 2021 |
| | | US 2022-0323369 A1 | 13 October 2022 |
| WO 2022-251665 A1 | | CA 3220689 A1 | 01 December 2022 |
| WO 2017-212006 A1 | 14 December 2017 | EP 3468608 A1 | 17 April 2019 |
| | | US 2019-0336611 A1 | 07 November 2019 |
| WO 2014-186366 A1 | 20 November 2014 | US 10792328 B2 | 06 October 2020 |
| | | US 2016-0082126 A1 | 24 March 2016 |
| | | US 2019-0240288 A1 | 08 August 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HIRKO et al.** *Curr, Med, Chem.*, 2003, vol. 10, 1185-1193
- **MERDAN et al.** *Adv. Drug. Deliv.Rev.*, 2002, vol. 54, 715-758
- **SPAGNOU et al.** *Biochemistry*, 2004, vol. 43, 13348-13386
- **FILONAND** ; **PHILLIPS**. *Biochim. Biophys/Acta*, 1997, vol. 1329, 345-356
- **REN et al.** *Gene Therapy.*, 2000, vol. 7, 764-768
- Remington's Pharmaceutical Science. Mack Publishing Company